# EUROPEAN PATENT APPLICATION

(11) **EP 3 168 596 A1**
(43) Date of publication of application: **17.05.2017**
(21) Application number: 15466015.3
(22) Date of filing: 03.12.2015
(51) Int. Cl.: G01N 3/08, A61F 2/82, G05D 23/20

(54) **TESTING THERMO-CHAMBER**

(30) Priority: 16.11.2015 CZ 201531765 U
(71) Applicant: Univerzita Karlova v Praze Lekarska Fakulta v Hradci Kralove, 500 38 Hradec Kralove (CZ)
(72) Inventor: Hanus, Josef, 500 08 Hradec Králové (CZ); Záhora, Jirí, 500 04 Hradec Králové (CZ); Bezrouk, Ales, 500 32 Hradec Králové (CZ); Kopecek, Martin, 503 31 Vysoká nad Labem (CZ)

(57) **Abstract**

A testing thermo-chamber, which is equipped with a bearing (1), heat exchanger (2) and a tension bar (4) for attaching the tested object. The inner room of the bearing ( 1 ) is equipped with a temperature sensor ( 3 ), the heat exchanger (2) being connected to more heating and cooling circuits (6) with multiport control valves (9), interconnected to thermostats (10) and by-passing branches (11), temperature sensor (3) is connected to the temperature regulator (12 ) further connected to thermostats (10) and a control unit (13) interconnected with multiport control valves (9).

## Description

### The technology

The suggested technical solution involves a testing thermo-chamber intended to be used for testing temperature-sensitive materials or objects, especially stents, to determine their characteristics and behaviour at various temperatures. The testing thermo-chamber consists of housing with a heat exchanger and a tension-bar prepared for attaching the tested object. The inner space of the housing is equipped with a temperature sensor.

### A present state of arts

So far the stents have been tested without regard to the testing environment. This means that it is impossible to ascertain the behaviour of the material and the overall behaviour of the stent after it has been introduced to the patient's body. A definite improvement of this situation would be a testing air thermo-chamber under the Czech norm CZ 20274 U1. Such a chamber would consist of a housing connected to the air circulation circuit which itself would be equipped with a heating and cooling unit. Simultaneously, the air-circulation circuit would be connected to the control element connected to the temperature sensor placed inside the housing. In the housing, a device for attaching the object tested is mounted and connected to the tension measuring device. However, this testing thermo-chamber can only be used for lower temperature ranges inside the housing and smaller temperature fluctuations.

The goal of this technical solution is to create precisely such a testing thermo-chamber that will enable, under testing conditions for tensile material such as stents, better adaptability to the real environment in the patient's body. This technical solution seeks to enable a wider range of temperatures and faster changes in temperature inside the housing of the thermo-chamber.

### The essence of the technical solution

The goal has been achieved by constructing a thermo-chamber in which the heat exchanger is connected to more heating and cooling circuits with engaged multiport control valves connected to the heat exchanger intake on one side and the outlet from the heat exchanger on the other side. The multiport control valves are connected to the thermostats and by-pass piping and housing equipped with a sensor connected to the heat regulator, which itself is further connected to the thermostats and a control unit linked up with multiport control valves.

The control unit can be linked up with a computer.

The tested object, for example a stent, is placed in the housing and attached to the tension bar connected with the tension device. In the housing there is also a heat exchanger connected to the multiport valves, among which are thermostats and by-pass branches for each heating and cooling circuits. The control by-pass valves are connected via control branching with the control unit, which itself is connected to the temperature regulator interconnected with the sensor located inside the housing. The temperature regulator is also interconnected with thermostats for the purpose of controlling and setting up temperature.

### Overview of figures and drawings

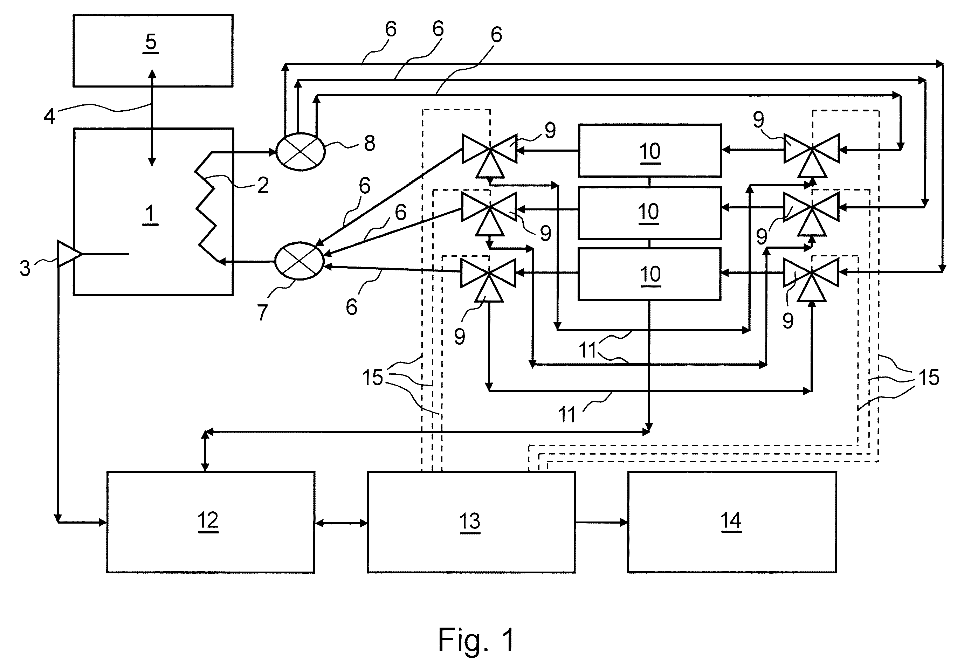

Schematic drawing of the testing thermo-chamber for the suggested technical solution - Figure No. 1.

### Examples of the technical solution

The testing thermo-chamber is equipped with a housing 1 inside which a tension bar 4 is placed and connected to the tension device 5 which is further equipped with a measuring device for measuring the lengthening of the tested material. On the opposite side of the tension device 5 a tension bar is placed 4 enabling testing material to be attached. The inner space of the housing 1 is equipped with a heat exchanger 2 which has an inlet mixer 7 and outcome mixer 8 into which three heating and cooling circuits 6 are connected. The circuits are also connected to the multiport control valves 9 - in this case three-port control valves. Each heating and cooling circuit 6 is equipped with a thermostat 10 placed between the multiport control valves 9. The multiport control valves 9 are connected to the by-pass branching 11 which enables the flow of the heating or cooling medium out of the heat exchanger 2. This solution enables the relevant thermoregulation circuit to be kept at the desired temperature. The housing 1 also contains a thermo sensor 3 connected to the regulator 12 which is linked to the thermostats 10 of the cooling circuits 6 and control unit 13 connected via control branches 15 with the multiport control valves 9. The control unit 13 is further connected to the computer 14.

During measuring, for example measuring the lengthening of a stent, the tested object is attached to the tension bar 4 and with the help of a tension device 5 undergoes tensing; its lengthening is measured and read on the measuring device. The heat exchanger 2 heats the inner room of the housing and in the process the hot or cold medium is brought through the input mixer 7 to the exchange part and goes out through the output mixer 8. By connecting these mixers to three heating and cooling circuits 6 we can easily change the temperature of the in-going medium to the exchanger 2, and most of all make a sharp change in temperature. The regulation of the in-coming hot or cold medium is done by multiport control valves 9, in this case three-port valves; their opening and closing is enabled via signals from the control unit 13 receiving the signal from the temperature regulator 12 connected to the temperature sensor 3 located inside the bearing 1. According to the data from the temperature regulator 12 the heating or cooling elements of the thermostat 10 are being heated or cooled. To achieve sufficiently rapid sharp changes in temperature inside the bearing 1 the medium in the heating and cooling circuits 6, which by the setting of the multiport valves 6 are not interconnected with the heat exchanger 2, is flowing through thermostats 10 and by-pass branches 11 so that it can keep the temperature of the medium identical to the relevant thermostat 10 through which this medium is flowing.

## Claims

1. A testing thermo-chamber, which is equipped with a bearing (1), heat exchanger (2) and a tension bar (4) for attaching the tested object, the inner room of the bearing (1) is equipped with a temperature sensor (3), **characterized in that**, the heat exchanger (2) being connected to more heating and cooling circuits (6) with multiport control valves (9), interconnected to thermostats (10) and by-passing branches (11), a temperature sensor (3) is connected to the temperature regulator (12), further connected to thermostats (10) and a control unit (13) interconnected with multiport control valves (9).

2. The testing thermo-chamber as described in the Required Protection 1, **characterized in that** the control unit (13) is connected to the computer (14).
